# FASCICULE DE BREVET EUROPEEN

(11) **EP 1 070 256 B1**
(45) Date de publication et mention de la délivrance du brevet: **22.09.2004**
(21) Numéro de dépôt: 99913389.5
(22) Date de dépôt: 12.04.1999
(51) Int. Cl.: G01N 33/552, G01N 33/551

(54) **FIXATION D'UNE MOLECULE BIOLOGIQUE SUR UNE SURFACE D'UN SUPPORT**
BEFESTIGUNG EINES BIOLOGISCHEN MOLEKÜLS AUF DER ÖBERFLACHE EINES TRÄGERS
METHOD FOR FIXING A BIOLOGICAL MOLECULE ON A SUPPORT SURFACE

(30) Priorité: 10.04.1998 FR 9804879
(43) Date de publication de la demande: 24.01.2001
(73) Titulaire: BIO MERIEUX, 69280 Marcy l'Etoile (FR)
(72) Inventeur: MALLET, François, F-69100 Villeurbanne (FR); THERETZ, Alain, F-69130 Ecully (FR); PEROUZEL, Eric, F-75005 Paris (FR); HEBRARD, Christine, 69540 Irigny (FR)
(74) Mandataire: Guerre, Dominique
(86) Numéro de dépôt international: PCT/FR1999/000848
(87) Numéro de publication internationale: WO 1999/053321

(56) Documents cités:
- EP-A- 0 272 792
- EP-A- 0 368 208
- EP-A- 0 482 571
- EP-A- 0 874 242
- WO-A-91/02980
- US-A- 5 492 814

## Description

La présente invention concerne un procédé de fixation d'un molécule biologique, tel qu'une protéine recombinante issue d'un antigène, un anticorps, une enzyme, mais également de peptides de synthèse et de PNA (peptide nucleic acids), sur la surface d'un support constitué de silice ou d'oxydes métalliques ; elles concernent également les supports ainsi réalisés et les utilisations qui peuvent être faites d'un tel support.

Il existe de nombreuses méthodes de fixation de molécules biologiques sur les supports de silice. Toutes passent par une étape de silanisation de la silice donnant à la surface les propriétés ou les fonctions recherchées. Cette fonctionnalisation permet ensuite de fixer ces composés par adsorption, couplage covalent ou complexation.

Premièrement, il y a la technique d'adsorption. Une telle technique est par exemple décrite dans les demandes de brevet EP-A-0.368.208 et WO-A-91/O2980. Dans ce cas, les protéines peuvent s'adsorber sur un support par plusieurs voies d'interactions (1). Les principales sont les interactions hydrophobes, polaires et ioniques. Ces interactions sont fonction à la fois du support, de la protéine et du milieu considérés.

L'adsorption hydrophobe s'effectue par une méthode d'adsorption sur support de silice plan employée au laboratoire (2) qui utilise les interactions entre une surface silanisée avec de longues chaînes alkyles et une ou plusieurs zones hydrophobes de la protéine.

Néanmoins, la fixation en terme d'orientation des éléments sur les chaînes alkyles n'est pas du tout uniforme, et seule une petite partie des éléments sera réellement disponible pour des réactions ultérieures, ce qui peut limiter la sensibilité.

Deuxièmement, il a y la technique de couplage. A ce propos, il existe également les méthodes de fixation covalente qui utilisent la silice fonctionnalisée par des silanes se terminant par des fonctions de type : amine, thiol ou époxy. Ces fonctions réagissent sur les groupements chimiquement réactifs des acides aminés accessibles de la protéine. La covalence s'effectue alors directement ou par l'intermédiaire d'agents de couplage.

La fixation de protéine sur un support par covalence est irréversible et s'effectue généralement sur plusieurs fonctions accessibles d'une protéine. Il en résulte parfois une inhibition de l'activité de la molécule.

Par exemple, la demande de brevet EP-A-0.874.242 concerne la silanisation d'un support (oxyde de silice) sur lequel peuvent être fixées de manière totalement aléatoire des molécules biologiques. La fixation s'effectue en plusieurs étapes. Premièrement, l'activation des silanols par hydrolyse des ponts oxygènes. Deuxièmement, la liaison entre les silanols et une molécule de fixation intermédiaire, qui peut recevoir un ligand.

Il y a donc au moins deux étapes, ce qui allongent la durée de réaction et n'est pas toujours compatible avec de bons résultats biologiques ou autres, ce qui complexifie la fixation du ligand et ce qui ne permet pas l'orientation du ligand. La fixation est alors aléatoire sur la molécule de fixation intermédiaire.

La demande de brevet EP-A-0.272.792 a sensiblement les mêmes inconvénients que la demande décrite ci-dessus, puisqu'elle propose de fixer, toujours de manière aléatoire, des antigènes sur des particules. Là encore, il y a une deuxième étape intermédiaire qui consiste en l'apport de bromure de cyanogène qui, par des liaisons covalentes, va associer indirectement les particules et les antigènes.

Ainsi ces deux demandes de brevet EP-A-0.874.242 et EP-A-0.272.792 sont donc très éloignées de l'un des objectifs principaux de la présente invention. Cet objectif consiste en l'orientation des molécules biologiques fixées sur le support.

Néanmoins, il est possible de fixer sélectivement de façon orientée une protéine possédant un « tag », par exemple lysine, tel que décrit dans la demande de brevet, déposée le 20 juin 1997 sous le numéro FR97/08055 par le demandeur.

Pourtant, il n'est pas question dans ce document de fixation en une seule étape sur un support constitué de silice ou d'oxyde métallique.

Troisièmement, il y a la technique de la chélation. Ainsi une molécide biologique peut également être fixée sélectivemem par chélation sur un support, selon un principe connu de l'homme du métier, tel que celui employé pour la purification des protéines par le procédé IMAC (Immobilized Metal ion-Affinity Chromatography) sur des résines (3, 4). Une telle protéine possède un marquage, dit « tag », riche en histidines. La difficulté de la méthode consiste à générer un support présentant des ions divalents nécessaires à la chélation. Une telle approche est décrite dans la demande de brevet, déposée le 16 avril 1997 sous le numéro FR97/04923 par le demandeur. Selon cette invention, un procédé de fixation de matériel biologique permet d'optimiser la complexation de ce matériel avec un complexe métallique, tout en diminuant, voire éliminant, toute réaction secondaire d'adsorption dudit matériel sur le complexe métallique. A cette fin, le procédé de fixation d'un matériel biologique, de l'invention, utilise un composé ligand de coordination, ou un composé complexe obtenu à partir de ce dernier, ledit composé ligand étant sous forme microparticulaire ou sous forme linéaire et constitué par au moins un polymère particulaire ou linéaire, avec une surface apparente hydrophile, et des groupes complexants libres, fixés de manière covalente.

L'objet de la présente invention est de permettre la fixation d'une molécule biologique, tel qu'une protéine recombinante issue d'un antigène, un anticorps, une enzyme, mais également de peptides de synthèse et de PNA (peptide nucleic acids) sur des supports de silice ou d'oxydes métalliques. Cette immobilisation utilise des interactions entre les silanols de la silice ou les fonctions alcools des oxydes métalliques et une zone spécifique de la protéine. Les propriétés particulières du support biospécifique ainsi créé sont principalement une bonne orientation des molécules biologiques ou ligands à la surface et un support engendrant de très bons rapports de signal sur bruit de fond. En outre, ce type d'immobilisation est simple et stable dans le temps.

A cet effet la présente invention concerne un procédé de fixation d'une molécule biologique sur la surface d'un support constitué de silice ou d'oxyde métallique, la molécule comportant un site spécifique de liaison, caractérisé en ce qu'il consiste à :
- fonctionnaliser la surface du support en nettoyant, par l'intermédiaire d'au moins un solvant ou un plasma d'oxygène ou tout autre procédé permettant la formation de groupements alcool au niveau de la surface du support, pour la rendre hydrophile,
- mettre en contact la molécule biologique directement sur ladite surface, et
- fonctionnaliser ledit support par la fixation du site spécifique de liaison de la molécule biologique sur au moins l'un des groupements alcool porté par la surface du support, ce qui permet l'orientation de la molécule pour une meilleure réactivité.

Selon un mode préférentiel d'utilisation, le procédé comporte, avant la mise en contact, une étape d'immersion de la surface fonctionnalisée dans un mélange sulfochromique.

Dans tous les cas de figure, le site spécifique de liaison de la molécule biologique comporte les caractéristiques suivantes :
- il s'agit d'une séquence en acides aminés rapportée, c'est-à-dire ajoutée à la séquence originale de la molécule biologique,
- cette séquence est introduite en un lieu privilégié de la séquence originale où elle est exposée de façon pertinente vis-à-vis de sa fonction ou de ses propriétés, et
- elle contient notamment des acides aminés d'intérêt vis-à-vis de la fonction recherchée.

Selon un premier mode de réalisation, le site spécifique de liaison de la molécule biologique est un site riche en histidines et ses dérivés, tel qu'un site contenant une densité suffisante d'histidines, notamment supérieure ou égale à 25%, et préférentiellement supérieure ou égale à 33%).

Plus précisément, selon un deuxième mode de réalisation, la molécule biologique et le site spécifique de liaison constituent une protéine recombinante comportant au moins deux histidines adjacentes, appelées « tag ».

Notamment, selon un troisième mode de réalisation, le site spécifique de liaison de la molécule biologique comporte au moins quatre histidines, et préférentiellement six histidines adjacentes.

Quel que soit le cas de figure, le site spécifique est situé au sein de la protéine recombinante ou préférentiellement à son extrémité N-terminale ou C-terminale.

La mise en contact s'effectue pendant 30 à 60 minutes.

Plus précisément, la mise en contact s'effectue pendant 45 minutes.

L'étape d'immersion s'effectue pendant 5 à 30 minutes.

Plus précisément, l'étape d'immersion s'effectue pendant 15 minutes.

Le procédé s'effectue à un potentiel hydrogène (pH) optimal en fonction des caractéristiques physico-chimiques de la molécule biologique et du site spécifique de liaison, préférentiellement compris entre 6 et 7,5.

La présente invention concerne également une surface d'un support fonctionnalisée par le procédé ci-dessus décrit.

Dans un mode préférentiel de réalisation, la surface est plane.

Dans un autre mode de réalisation, la surface est constituée par une bille.

La présente invention concerne encore l'utilisation d'une surface fonctionnalisée d'un support, tel que décrit ci-dessus, ou fabriquée par le procédé, décrit plus haut, caractérisée en ce qu'elle consiste à effectuer des tests pour détecter, dans un liquide à analyser, la présence de toute molécule biologique capable de se fixer, par un site spécifique de liaison, sur le support.

Dans un mode préférentiel d'utilisation, une surface fonctionnalisée d'un support, tel que décrit ci-dessus, ou fabriquée par le procédé, décrit plus haut, est utilisé pour effectuer des tests immunologiques pour détecter, dans un liquide à analyser, la présence d'anticorps spécifiques d'une protéine naturelle, par exemple la protéine P24, par liaison spécifique, de la même manière qu'avec la protéine naturelle, sur au moins une protéine recombinante, par exemple la protéine RH24 ou R24.

Cette protéine P24 est une protéine recombinante de la capside du virus de l'immunodéficience humaine de type 1 (VIH-1).

Les figures ci-jointes sont données à titre d'exemple indicatif et n'ont aucun caractère limitatif quant à la portée exacte de la présente invention.

La figure 1 représente un graphique comparatif de la densité optique (DO) obtenue sur un test ELISA de RH24 et R24 absorbées sur différents supports.

La figure 2 représente un graphique comparatif du coefficient signal / bruit du système protéine absorbée RH24 et R24 sur différents supports.

La figure 3 représente une courbe d'effet du pH sur l'absorption de RH24 sur silice.

La figure 4 représente une courbe de dosage des anticorps monoclonaux 15F8C7 par SFMIA (Scanning Force Microscopic ImmunoAssay).

Enfin, la figure 5 représente une courbe de dosage des anticorps polyclonaux de lapin par SFMIA.

Un support plan d'oxyde de silicium ou silicee et une protéine recombinante de la capside du virus de l'immunodéficience humaine de type 1 (VIH-1) sont utilisés comme modèle. Ce système permet l'exploitation de la microscopie à force atomique (AFM) en tant que méthode d'analyse immunologique ou SFMIA (2).

La protéine considérée ici est la RH24. Elle est obtenue en utilisant les méthodes classiques de biologie moléculaire et d'expression eucaryote (5). Celle-ci est quasiment semblable à la R24 (immunogénicité identique) mais est beaucoup plus facilement purifiée grâce à la présence d'une petite séquence d'acides aminés supplémentaires. Cette série d'aminoacides, appelée « tag », contient en particulier six histidines consécutives. L'affinité d'un tel motif moléculaire pour certains ions métalliques est extrêmement forte. Cette propriété permet une purification aisée avec la technique IMAC (chromatographie d'affinité métal-chélate). La protéine de contrôle sans le « tag » est la R24. Elle est obtenue de la même façon que la RH24 mais elle est purifiée par immunoaffinité.

Les sites spécifiques de liaison peuvent se présenter sous la forme de suites desdits acides aminés, identiques ou différents, contigus ou non, mais voisins.

La définition du « tag » est la suivante :
1/ il s'agit d'une séquence en acides aminés rapportée, c'est à dire ajoutée à la séquence originale (protéique, peptidique, PNA),
2/ cette séquence est introduite en un lieu privilégié de la séquence originale où elle est exposée de façon pertinente vis-à-vis de sa fonction (ou de ses propriétés, par exemple chélation, couplage, interactions avec un support particulier, etc), en particulier les extrémités N-terminale et C-terminale des protéines recambinantes ou des peptides de synthèse ou des PNA, et
3/ elle contient notamment des acines aminés d'intérêt vis-à-vis de la fonction recherchée, dans notre cas des histidines, distribués à l'intérieur de la séquence soit de façon contiguë (notamment deux histidines continuës, préférentiellement six histidines contiguës), soit avec une densité suffisante (notamment 25%, préférentiellement supérieure ou égale à 33%).

Avantageusement, ledit site de liaison comprend de trois à dix acides aminés, contigus ou non contigus, pour une structure primaire ordonnée du matériel protéique comprenant au moins trente acides aminés. A titre de variante préférée, il consiste en au moins deux, préférentiellement au moins trois, et préférentiellement au moins six résidus d'histidine, plus particulièrement, ces résidus sont contigus.

Le ou les sites décrits ci-dessus peuvent se trouver à un endroit quelconque de la structure primaire du matériel protéique. De préférence, il est situé sur l'extrémité N-terminale ou C-terminale du matériel protéique.

La silice, disponible sous de nombreuses formes (surfaces planes, billes, fibres, etc.), est un composé abondamment utilisé en micro-électronique. Or, la recherche en diagnostic médical s'intéresse de plus en plus à des systèmes dits « intégrés » (DNA chips, biochips,...) comprenant des puces sensibles à des molécules biologiques. De telles technologies allieraient sensibilité, rapidité et miniaturisation.

De plus, il est possible d'obtenir des surfaces de silice dont la rugosité n'excède pas quelques Angströms. Cette propriété permet alors l'utilisation d'un moyen de détection et d'analyse extrêmement puissant: la microscopie à force atomique (AFM). La microscopie à force atomique réalise aussi bien l'observation des surfaces fonctionnalisées par des molécules biologiques que des immunoessais très sensibles (2).

Il est apparu que la RH24 possède un affinité naturelle pour les supports de silice. Cette propriété provient d'une interaction directe forte entre la surface d'un support et la séquence poly(histidine).

La présente invention vise donc à fixer une protéine recombinante sur la silice par l'intermédiaire de sa séquence d'histidines. Cette technique permet aussi d'orienter une telle protéine sur la surface. En effet, si la zone exposée de la molécule est un site de reconnaissance, alors celle-ci est d'autant plus réactive (site actif d'enzyme ou épitope immunodominant par exemple).

Plus précisément, l'invention concerne la fixation orientée de la RH24 et de toute protéine recombinante sur silice. Il s'inscrit donc dans cette tendance. Des informations complètes sur la protéine R24 et ses recombinants RH24 et RH24K peuvent être trouvées dans une demande de brevet précédente du demandeur, déposée le 20 juin 1997 sous le numéro FR97/08055. Le contenu de cette demande est incorporé à la présente invention.

L'invention concerne une voie de fixation directe, sans silanisation de la silice, pour permettre la liaison entre la silice et la protéine recombinante.

La démarche utilise l'affinité particulière du « tag » de la RH24 pour la silice hydrophile. Cette technique de fixation est nouvelle et extrêmement simple et robuste. Elle est l'objet de la protection recherchée par la présente invention.

L'intérêt de cette immobilisation selon la seconde démarche est à trouver dans deux techniques majeures, d'une part, l'ELISA pour sa simplicité et sa robustesse, et d'autre part, le dosage immunologique par AFM (SFMIA) pour sa finesse et sa sensibilité.

### Exemple 1 : Fabrication du support biospécifique :

A titre de comparaison, l'adsorption de la R24 et de la RH24 sur différents supports de silice a été testée. Trois types de supports plan de silice ont été utilisés :
- une silice nue partiellement hydrophobe car non nettoyée et non hydrolysée par le mélange sulfochromique,
- une silice très hydrophile car nettoyée et hydrolysée par le mélange sulfochromique, et
- une silice très hydrophobe fonctionnalisée avec du n-octadécyldiméthylméthoxysilane.

Les supports utilisés ici sont des disques de silicium (disque poli de 4" de chez ACM (France), type N dopé P, épaisseur 525 micromètres (µm), orientation <100>)

Dans un premier temps, ces disques sont clivés en plaques carrées de 0,64 cm².

### 1) Silice nue partiellement hydrophobe:

Les plaques clivées présentent à leur surface, une couche d'oxyde de silicium, native ou obtenue par les différents procédés connus de l'homme de l'art. Cette couche d'oxyde présente, entre autres, des ponts siloxanes hydrophobes et elle peut être polluée par les contaminants atmosphériques.

Une silice de ce type est partiellement hydrophobe.

### 2) Silice très hydrophile :

Afin d'obtenir des supports de silice hydrophiles, propres et de taille désirée, les plaques subissent le traitement suivant :
- nettoyage de la surface : les plaques de silicium sont lavées avec divers solvants ; dans l'ordre sont utilisés : eau, éthanol, acétone, dichlorométhane, toluène, et
- hydrolyse des ponts siloxanes en silanols : les supports de silice sont immergés pendant 15 minutes dans un mélange sulfochromique (solution PROLABO saturée en Chrome (VI) oxyde dans l'acide sulfurique à 95%). Ces surfaces sont abondamment rincées à l'eau et passées au bain à ultrasons ; une fois les plaques séchées à l'azote, elles sont utilisées rapidement pour limiter une rapide recontamination.

La caractéristique principale d'une silice propre est sa grande hydrophilie. Celle-ci peut être vérifiée par la mesure d'angle de contact (angle entre la tangente au bord de la goutte d'eau et la surface du support) qui doit être inférieur ou égal à 10°.

### 3) Silice hydrophobe :

Les plaques sont nettoyées comme décrit ci-dessus (paragraphe 2).

Puis, la silice est rendue hydrophobe par mise en contact de n-octadécyl-diméthylméthoxysilane, silane portant une longue chaîne alkyle, à 2 % dans le toluène pendant trois heures à température ambiante. Les plaques sont ensuite lavées abondamment à l'eau, nettoyées aux ultrasons et stabilisées pendant deux heures à 120 °C.

### 4) Adsorption des protéines sur les différents supports décrits :

Dans le cas de la R24 ou de la RH24, il est possible d'effectuer une adsorption sur un support hydrophile (support décrit en paragraphe 2) ou hydrophobe (supports décrits dans les paragraphes 1 et 3).

Dans le cas d'une adsorption sur un support hydrophile, il n'y a pas besoin de fonctionnaliser la surface de silice traitée précédemment, celle-ci est considérée comme déjà fonctionnalisée.

Cette technique n'est habituellement jamais utilisée sur la silice nue hydrophile. En effet, ces interactions sont généralement trop fragiles (très sensibles au pH et aux variations de force ionique). Cependant, dans le cas de la RH24, ou de tout autre « ligands » tels que définis précédemment, la présence du « tag » rend cette adsorption extrêmement intéressante, car très facile à mettre en oeuvre.
* 40 microlitres de RH24 (ou de R24 comme témoin) diluée dans le PBS, sont incubés pendant 45 minutes à température ambiante sur silice hydrophile.
   La RH24 possède une forte affinité pour la silice. L'intérêt de cette affinité est sa spécificité envers la RH24 par comparaison avec la R24. A partir de ce point, l'analyse qui suit tente de mieux comprendre le rôle du « tag ».
* 40 microlitres de solution de protéine dans le PBS sont incubés durant 2 heures à température ambiante sur les plaques silanisées. Puis elles sont rincées par le PBS-Tween (Tween à 0,05 % dans le PBS).

### Exemple 2 : Tests du support biospécifique :

La présence est l'orientation de la R24 et de la RH24 sont révélées par deux types d'immunoessais : ELISA et SFMIA.

La présence de la R24 ou de la RH24 à la surface du support est évaluée au moyen d'anticorps. Des solutions polyclonales ou monoclonales sont utilisées. Une goutte de 40 µl d'anticorps en solution dans le mélange PBS-TWEEN-BSA [0.05% de BSA (w/w) dans PBS-TWEEN] de concentration désirée est déposée durant 1 heure à 37°C sur une plaque. La BSA permet comme le TWEEN de limiter les interactions non spécifiques. Le carré de silicium est ensuite lavé avec du PBS-TWEEN-BSA afin d'éliminer au maximum les anticorps adsorbés sur le support et non fixés sur la protéine. Les plaques sont séchées à l'air comprimé.

### 1) Test enzymatique (ELISA) :

La quantité d'anticorps biotinylés, capturée par la protéine recombinante, est évaluée par un test enzymatique. Un complexe phosphatase alcaline (PAL)-Streptavidine est fixé en premier. Pour cela, on fait réagir à 37°C durant 45 minutes, 40 µl d'une solution de Streptavidine-PAL à 4 g/ml dans PBS-TWEEN-BSA.

Puis la présence de l'enzyme est révélée par addition d'un substrat colorimétrique, le p-nitophenyl phosphate (pnPP). Une plaque est immergée dans 0,8 ml de solution pnPP à 2 mg/ml dans son tampon. On laisse réagir l'enzyme sur son substrat 45 minutes à 37°C. Puis la réaction est interrompue par l'ajout de 0,8 ml de soude 1 N.

Le dosage colorimétrique à 405 nm est effectué sur 100 µl de solution dans des microplaques pour lecteur AXIA. La DO obtenue est liée à la concentration de protéine à la surface.

### 2) Test SFMIA :

La quantité d'anticorps biotinylés, capturée par la protéine recombinante, sur le support est évaluée au moyen d'un marqueur topographique d'AFM :
* 40 µl d'une solution à 5% (V/V) de billes magnétiques couvertes de streptavidines et d'un diamètre de 50 nm sont déposés durant 1 heure sur le support. Afin d'aider la réaction de surface, les supports sont placés sur des aimants puissants en terres rares. Puis les plaques sont rincées à l'eau et séchées à l'air comprimé.
Le nombre de billes magnétiques à la surface est proportionnel à la quantité de protéines retenue sur le support. Ce nombre de billes est quantifié par AFM.

### Exemple 3 : Comparaison des modes de fixation de la R24 et de la RH24 :

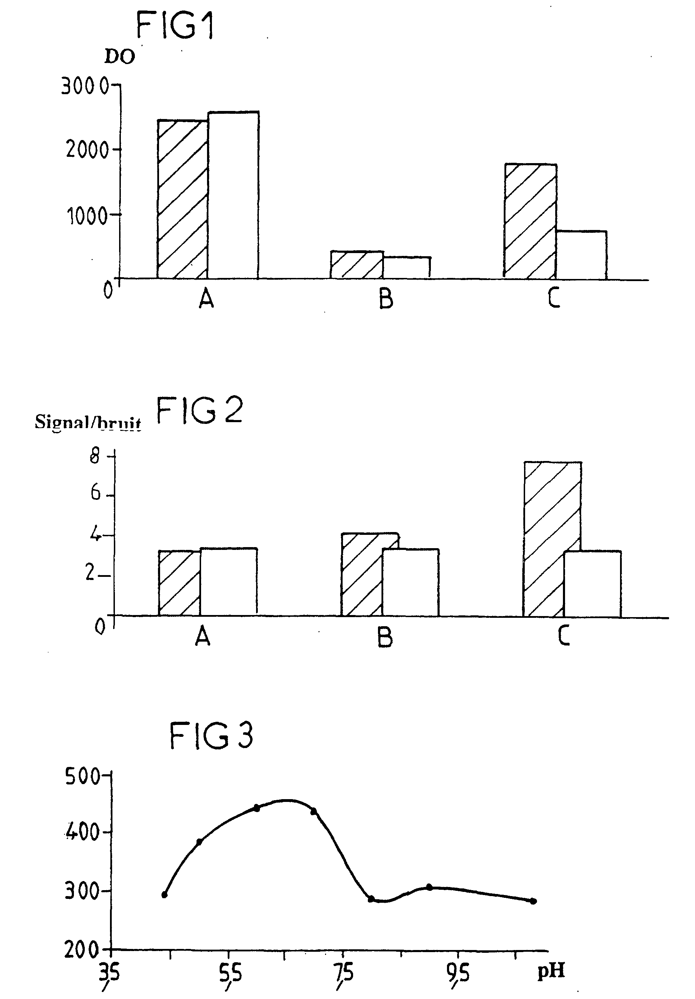

Pour des concentrations de 10 µg/ml en protéines révélées en ELISA avec l'anticorps 13B5 à 1 µg/ml dans les conditions standards, les résultats de la figure 1 sont obtenus.

Cet anticorps a fait l'objet d'un dépôt de demande de brevet français, le même jour que cette demande de brevet, par la demanderesse. Le titre de cette autre demande est : « Ligand peptidique présentant une affinité spécifique vis à vis de la protéine P24 du rétrovirus HIV ».

Seul le support de silice hydrophile montre une différence de signal significatif entre la R24 et la RH24. Ceci montre que le « tag » de la RH24 joue un rôle particulier dans l'adsorption hydrophile.

D'autre part, si le graphique des rapports signal/bruit est tracé, les résultats obtenus sont présentés à la figure 2.

Outre la spécificité de l'adsorption sur un support hydrophile, ce support permet d'obtenir un coefficient signal/bruit excellent. Ceci provient probablement du fait que l'adsorption des anticorps se fait peu sur un support hydrophile (le Fc des anticorps est plutôt hydrophobe).

La limitation du bruit de fond est un facteur essentiel pour réaliser des systèmes sensibles de détection. Donc, même si le signal brut obtenu est un peu inférieur à celui d'une surface hydrophobe, le gain en bruit de fond en fait un système potentiellement plus intéressant.

### 1) pH et force ionique :

L'affinité de la RH24 pour la silice est remarquable car elle est très peu sensible à la force ionique du milieu environnant. Ainsi, des rinçages à l'eau ou avec une solution de NaI 9 M n'ont aucun effet sur la surface. La RH24 une fois fixée est donc fortement liée au support.

La RH24 a été diluée à 1 µg/ml dans différents tampons phosphate dont les pH varient entre 4 et 11. Puis elle est adsorbée et révélée par un test ELISA standard au polyclonal de lapin à 1 µg/ml.-Les conditions optimales d'adsorption obtenues se situent entre 6 et 7 (voir la figure 3).

Sachant que le point isoélectrique de la protéine est de 6,05, que le pKa d'une, séquence polyhistidine est de 6 et que le pKa des silanols est compris entre 4 et 7, ce résultat est cohérent avec plusieurs modes d'interaction possibles (liaisons induites entre le cycle aromatique de l'histidine et les silanols, liaisons hydrogènes entre les silanols et les groupements donneurs ou accepteurs d'électrons, liaisons électrostatiques entre des silanols déprotonés (-SiO⁻) et des noyaux imidazoles chargés positivement à pH inférieur ou égal à 6. Le "tag" présentant une surconcentration locale de ces différents types d'interaction, ceci permet d'expliquer l'affinité et la solidité de la liaison.

### 2) Force de liaison :

Afin de démontrer que le « tag » a effectivement une grande affinité pour le support, une adsorption de la RH24 en compétition avec un polypeptide de séquence équivalente au « tag » (MRGSHHHHHHSVDES) a été effectuée. Pour cela, une solution à 3,7.10-7 mol/l en RH24 (10 µg/ml) et à 3,7.10-4 mol/l en polypeptide est préparée (1 molécule de RH24 pour 1000 molécules de polypeptide). Puis la plaque est incubée avec ce mélange et révélée en ELISA avec le monoclonal 13B5D10 à 1 µg/ml. Les résultats suivants sont obtenus :

**Tableau III :**

| Densité obtenue pour un mélange « tag »-RH24 | |
|---|---|
| Réaction | Densité optique (bruit de fond soustrait) |
| Mélange polypeptide-RH24 | 110 |
| Protéine seule | 780 |

Le signal décroît de plus de 85 % en présence du polypeptide, ce qui suggère que la séquence, en occupant les sites de fixation empêche la RH24 de se fixer sur son support.

Ce résultat a également été obtenu par une compétition avec la pyridine mais il a fallu une solution 0,11 mol/l pour réduire le signal de plus de 70 % (1 molécule de RH24 pour 300 000 molécules de pyridine). Sachant que l'affinité de la pyridine pour la surface de silice est considérée comme très forte, il est manifeste que le « tag » de la RH24 possède une affinité excellente pour le support. De tels résultats n'ont pas pu être obtenus avec l'imidazole. Ceci montre que l'interaction forte entre la RH24 et la surface provient non seulement de la nature des groupements concernés mais également de leur nombre sur le « tag ». Cet effet coopératif de liaisons multiples explique la force de cette liaison.

Tous ces résultats permettent de conclure que l'affinité de la RH24 pour la silice est forte. Le modèle des liaisons multiples entre le « tag » et les silanols a été démontré de manière indirecte par compétition.

### 3) Orientation :

La sensibilité du système de détection de la RH24 sur support de silice est évaluée au moyen d'un SFMIA.

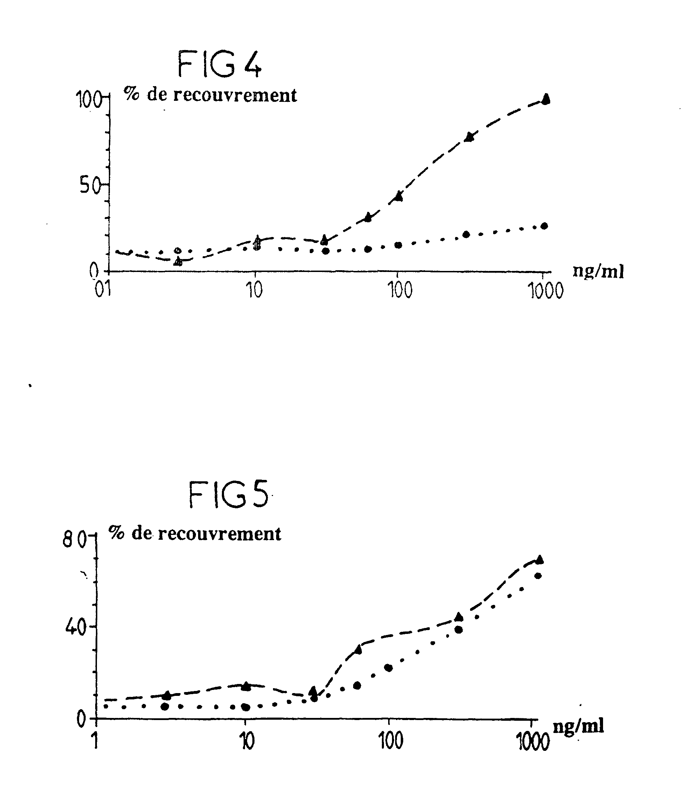

Une solution de protéine à 10 µg/ml est incubée sur des plaques. Ces plaques sont révélées par SFMIA en utilisant des anticorps polyclonaux et monoclonaux (15F8C7) marqués par des particules magnétiques. La quantité de billes magnétiques couvrant la surface est évaluée grâce au système de traitement d'images. Les courbes de pourcentage de recouvrement en fonction de la concentration en anticorps utilisés sont ainsi tracées aux figures 4 et 5.

Les résultats obtenus en SFMIA montrent une différence importante de signal entre la R24 et la RH24 lorsqu'un anticorps monoclonal est utilisé.

Les monoclonaux utilisés n'étant pas dirigés à l'encontre du « tag », ils ont intrinsèquement la même réactivité vis-à-vis de la R24 et de la RH24. L'augmentation de signal concernant la RH24 peut provenir de deux phénomènes. La RH24 est plus concentrée superficiellement que la R24, ou la RH24 est orientée sur son support.

Si la RH24 est partiellement orientée sur son support, alors l'épitope de fixation de l'anticorps monoclonal est accessible plus fréquemment.

En fait, en cas de FIXATION ORIENTEE, l'épitope reconnu par le monoclonal est TOUJOURS accessible ou JAMAIS, alors que dans le cas d'une FIXATION ALEATOIRE, il ne sera accessible que PARFOIS. Il en résulte donc une augmentation du signal en immunoessai sur ce monoclonal. Cette amplification du signal n'aura pas lieu avec un polyclonal sur la RH24 car celle-ci n'a pas d'épitope immunodominant. Orienter une telle protéine sur un support n'a donc pas d'intérêt pour une solution polyclonale.

Si l'effet est dû à la concentration de surface, cela provient nécessairement d'une interaction spécifique engendrée par le « tag ». Cette interaction étant géographiquement localisée sur la protéine, elle est alors indissociable d'un effet d'orientation.

### 4) Conclusions :

Les expériences réalisées montrent clairement que la spécificité de l'interaction entre la silice et la RH24 provient du site spécifique de liaison ou « tag ».

L'immobilisation obtenue est peu sensible aux variations de force ionique et conserve ses propriétés immunogéniques sur une longue période. De plus, ce support donne de bien meilleurs rapports signal/bruit qu'un système par adsorption sur un support hydrophobe.

Les tests standards de dosages enzymatiques d'anticorps anti-R24 sont habituellement réalisés sur des supports (puits de polystyrène) où la protéine est adsorbée par interactions hydrophobes. Les limites de détection obtenues par adsorption sur silice hydrophile sont comparables à ces immunoessais.

En outre, le support plan utilisé se prête à l'utilisation de la microscopie à force atomique. Les tests SFMIA réalisés avec des billes magnétiques, tels que décrits dans la demande de brevet FR97/01313 déposée le 30 janvier 1997 au nom de la demanderesse, permettent d'effectuer des immunoessais de haute sensibilité.

La comparaison systématique entre le RH24 et la R24 pour les immunoessais a montré une similarité de comportement vis-à-vis d'un polyclonal et une sensible différence vis-à-vis des anticorps monoclonaux. L'orientation de la RH24 à la surface en est la meilleure explication.

### BIBLIOGRAPHIE

(1) Gautier, S., Aimé, J.P., Bouhacina, T., Attias, A.J., Desbat, B., *Langmuir* **12,** 5126 (1996).
(2) Perrin, A., Lanet, V., Theretz, A., *Langmuir* **13,** 2557 (1997).
(3) Porath J., Carlsson., Olsson., Belfrage J., *Nature*, **258,** 598 (1975).
(4) Porath J., *Trends Anal. Chem.,* **7,** 254 (1988)
(5) Cheynet, V., Verrier, B., Mallet, F., *Protéine Expr*. *Purif*. *L,* 367-372 (1993).

## Revendications

1. Procédé de fixation d'une molécule biologique sur la surface d'un support constitué de silice ou d'oxyde métallique, la molécule comportant un site spécifique de liaison, caractérisé en qu'il consiste à :
- fonctionnaliser la surface du support en nettoyant, par l'intermédiaire d'au moins un solvant ou un plasma d'oxygène ou tout autre procédé permettant la formation de groupements alcool au niveau de la surface du support, pour la rendre hydrophile,
- mettre en contact la molécule biologique directement sur ladite surface, et
- immobiliser sur ledit support la molécule biologique, au niveau de son site spécifique de liaison, par sa fixation sur au moins l'un des groupements alcool porté par la surface du support, ce qui permet l'orientation de la molécule pour une meilleure réactivité,
ledit site spécifique de liaison de la molécule biologique comportant les caractéristiques suivantes :
- il s'agit d'une séquence en acides aminés rapportée, c'est-à-dire ajoutée à la séquence originale de la molécule biologique, et
- cette séquence est introduite au niveau de la séquence originale en un emplacement où elle est exposée de façon à permettre son immobilisation sur un support.

2. Procédé, selon la revendication 1, **caractérisé en ce qu'**il comporte, avant la mise en contact, une étape d'immersion de la surface fonctionnalisée dans un mélange sulfochromique.

3. Procédé, selon l'une quelconque des revendications 1 et 2, **caractérisé en ce que** le site spécifique de liaison de la molécule biologique est un site riche en histidines et ses dérivés, tel qu'un site contenant une densité d'histidines, supérieure ou égale à 25%, et préférentiellement supérieure ou égale à 33%.

4. Procédé, selon l'une quelconque des revendications 1 à 3, **caractérisé en ce que** la molécule biologique et le site spécifique de liaison constituent une protéine recombinante comportant au moins deux histidines adjacentes, appelées "tag".

5. Procédé, selon l'une quelconque des revendications 1 à 4, **caractérisé en ce que** le site spécifique de liaison de la molécule biologique comporte au moins quatre histidines et préférentiellement six histidines adjacentes.

6. Procédé, selon la revendication 5, **caractérisé en ce que** le site spécifique est situé au sein de la protéine recombinante ou préférentiellement à son extrémité N-terminale ou C-terminale.

7. Procédé, selon l'une quelconque des revendications 1 à 6, **caractérisé en ce que** la mise en contact s'effectue pendant 30 à 60 minutes.

8. Procédé, selon l'une quelconque des revendications 1 à 7, **caractérisé en ce que** la mise en contact s'effectue pendant 45 minutes.

9. Procédé, selon la revendication 2, **caractérisé en ce que** l'étape d'immersion s'effectue pendant 5 à 30 minutes.

10. Procédé, selon l'une quelconque des revendications 2 ou 9, **caractérisé en ce que** l'étape d'immersion s'effectue pendant 15 minutes.

11. Procédé, selon l'une quelconque des revendications 1 à 10, **caractérisé en ce qu'**il s'effectue à un potentiel hydrogène (pH) permettant l'immobilisation orientée de la molécule biologique par fixation de son site spécifique de liaison au niveau du support, préférentiellement ce pH est compris entre 6 et 7,5.

12. Utilisation d'une surface d'un support fonctionnalisée par le procédé selon l'une quelconque des revendications 1 ou 11 pour la fixation orientée d'une molécule biologique.

13. Utilisation selon la revendication 12, **caractérisée par le fait que** ladite surface est plane.

14. Utilisation selon la revendication 12, **caractérisée par le fait que** ladite surface est constituée par une bille.

15. Utilisation selon la revendication 12, **caractérisée en ce qu'**elle consiste à effectuer des tests pour détecter, dans un liquide à analyser, la présence de toute molécule biologique capable de se fixer, par un site spécifique de liaison, sur le support.

16. Utilisation selon la revendication 12, **caractérisée en ce qu'**elle consiste à effectuer des tests immunologiques pour détecter, dans un liquide à analyser, la présence d'anticorps spécifiques d'une protéine naturelle, par exemple la protéine P24, par liaison spécifique, de la même manière qu'avec la protéine naturelle, sur au moins une protéine recombinante, par exemple la protéine RH24 ou R24.

## Patentansprüche

1. Verfahren zur Fixierung eines eine spezifische Bindungsstelle aufweisenden biologischen Moleküls auf der Oberfläche eines Trägers, welcher aus Siliciumdioxid oder einem metallischen Oxid gebildet ist, **dadurch gekennzeichnet, dass** das Verfahren die folgenden Schritte aufweist, nämlich:
- Funktionalisieren der Oberfläche des Trägers durch Waschen, mit Hilfe von zumindest einem Lösungsmittel oder einem sauerstoffhaltigen Plasma, oder durch jedes andere Verfahren, welches die Bildung von Alkoholgruppen im Bereich der Oberfläche des Trägers ermöglicht, um diese hydrophil zu machen,
- direktes In-Kontakt-Bringen des biologischen Moleküls mit der Oberfläche, und
- Immobilisieren des biologischen Moleküls an dem Träger im Bereich von dessen spezifischer Bindungsstelle, wobei das Molekül über zumindest eine der auf der Oberfläche des Trägers vorliegenden Alkoholgruppen derart fixiert wird, dass die Ausrichtung des Moleküls eine bessere Reaktivität ermöglicht,
wobei die spezifische Bindestelle des biologischen Moleküls die folgenden Merkmale aufweist:
- es handelt sich um eine angefügte Aminosäure-Sequenz, d.h. um eine Sequenz, die an die ursprüngliche Sequenz des biologischen Moleküls angehängt ist, und
- diese Sequenz ist im Bereich der ursprünglichen Sequenz an einer Stelle eingeführt, wo sie derart exponiert ist, dass sie die Immobilisierung an den Träger ermöglicht.

2. Verfahren nach Anspruch 1, **dadurch gekennzeichnet, dass** es vor dem In-Kontakt-Bringen den Schritt des Eintauchens der funktionalisierten Oberfläche in eine Schwefel-Chrom-Mischung aufweist.

3. Verfahren nach einem der Ansprüche 1 oder 2, **dadurch gekennzeichnet, dass** die spezifische Bindungsstelle des biologischen Moleküls eine Stelle ist, die reich an Histidinen und Histidin-Derivaten ist, sowie eine Stelle, die eine Histidindichte aufweist, die größer oder gleich 25 % und vorzugsweise größer oder gleich 33 % ist.

4. Verfahren nach einem der Ansprüche 1 bis 3, **dadurch gekennzeichnet, dass** das biologische Molekül und die spezifische Bindungsstelle ein rekombinantes Protein bilden, welches zumindest zwei nebeneinander liegende Histidine aufweist, die als "Tag" bezeichnet werden.

5. Verfahren nach einem der Ansprüche 1 bis 4, **dadurch gekennzeichnet, dass** die spezifische Bindestelle des biologischen Moleküls zumindest vier und vorzugsweise sechs nebeneinander liegende Histidine aufweist.

6. Verfahren nach Anspruch 5, **dadurch gekennzeichnet, dass** die spezifische Bindestelle innerhalb des rekombinanten Proteins und vorzugsweise an dessen N- oder C-terminalen Ende gelegen ist.

7. Verfahren nach einem der Ansprüche 1 bis 6, **dadurch gekennzeichnet, dass** das In-Kontakt-Bringen über einen Zeitraum von 30 bis 60 Minuten erfolgt.

8. Verfahren nach einem der Ansprüche 1 bis 7, **dadurch gekennzeichnet, dass** das In-Kontakt-Bringen über einen Zeitraum von 45 Minuten erfolgt.

9. Verfahren nach Anspruch 2, **dadurch gekennzeichnet, dass** der Schritt des Eintauchens über einen Zeitraum von 5 bis 30 Minuten erfolgt.

10. Verfahren nach einem der Ansprüche 2 oder 9, **dadurch gekennzeichnet, dass** der Schritt des Eintauchens über 15 Minuten erfolgt.

11. Verfahren nach einem der Ansprüche 1 bis 10, **dadurch gekennzeichnet, dass** es bei einem pH-Wert durchgeführt wird, der die gerichtete Immobilisierung des biologischen Moleküls durch die Fixierung von dessen spezifischer Bindestelle im Bereich des Trägers ermöglicht, wobei bevorzugt ist, wenn der pH-Wert bei 6 bis 7,5 liegt.

12. Verwendung einer funktionalisierten Trägeroberfläche für ein Verfahren nach einem der Ansprüche 1 oder 11 zu einer gerichteten Fixierung eines biologischen Moleküls.

13. Verwendung nach Anspruch 12, **dadurch gekennzeichnet, dass** die Oberfläche eben ist.

14. Verwendung nach Anspruch 12, **dadurch gekennzeichnet, dass** die Oberfläche durch eine Kugel gebildet ist.

15. Verwendung nach Anspruch 12, **dadurch gekennzeichnet, dass** sie darin besteht, Tests durchzuführen, um in einer zu analysierenden Flüssigkeit das Vorliegen des biologischen Moleküls zu detektieren, wobei das biologische Molekül über eine spezifische Bindestelle an den Träger fixiert werden kann.

16. Verwendung nach Anspruch 12, **dadurch gekennzeichnet, dass** sie darin besteht immunologische Tests durchzuführen, um in einer zu analysierenden Flüssigkeit das Vorliegen von spezifischen Antikörpern gegen ein natürliches Protein, bspw. das Protein P24 zu detektieren, und zwar durch spezifische Bindung über zumindest ein rekombinantes Protein, bspw. über das Protein RH24 oder R24, wobei die spezifische Bindung auf die gleiche Art und Weise erfolgt wie über das natürliche Protein.

## Claims

1. Method for attaching a biological molecule to the surface of a support consisting of silica or of metal oxide, the molecule comprising a specific binding site, **characterized in that** it consists in:
- functionalizing the surface of the support by cleaning, using at least one solvent or an oxygen plasma or any other process for forming alcohol groups on the surface of the support, in order to render said surface hydrophilic,
- bringing the biological molecule directly into contact with said surface, and
- immobilizing the biological molecule, at the level of its specific binding site, on said support by attaching it to at least one of the alcohol groups borne by the surface of the support, which allows the molecule to be oriented for better reactivity,
said specific binding site of the biological molecule comprising the following characteristics:
- it is an added amino acid sequence, i.e. added to the original sequence of the biological molecule, and
- this sequence is introduced into the original sequence at a place, where it is exposed in such a way as to allow its immobilization on a support.

2. Method according to Claim 1, **characterized in that** it comprises, before the bringing into contact, a step of immersion of the functionalized surface in a sulphochromic mixture.

3. Method according to either of Claims 1 and 2, **characterized in that** the specific binding site of the biological molecule is a site rich in histidines and derivatives thereof, such as a site containing a density of histidines higher than or equal to 25%, and preferably higher than or equal to 33%.

4. Method according to any one of Claims 1 to 3, **characterized in that** the biological molecule and the specific binding site constitute a recombinant protein comprising at least two adjacent histidines called "tag".

5. Method according to any one of Claims 1 to 4, **characterized in that** the specific binding site of the biological molecule comprises at least four adjacent histidines, and preferably six adjacent histidines.

6. Method according to Claim 5, **characterized in that** the specific site is located within the recombinant protein or preferably at its N-terminal or C-terminal end.

7. Method according to any one of Claims 1 to 6, **characterized in that** the bringing into contact is carried out for 30 to 60 minutes.

8. Method according to any one of Claims 1 to 7, **characterized in that** the bringing into contact is carried out for 45 minutes.

9. Method according to Claim 2, **characterized in that** the immersion step is carried out for 5 to 30 minutes.

10. Method according to either of Claims 2 and 9, **characterized in that** the immersion step is carried out for 15 minutes.

11. Method according to any one of Claims 1 to 10, **characterized in that** it is carried out at a hydrogen potential (pH) which allows the oriented immobilization of the biological molecule by attachment of its specific binding site to the support; preferably this pH is between 6 and 7.5.

12. Use of a surface of a support functionalized by the method according to either of Claims 1 and 11, for the oriented attachment of a biological molecule.

13. Use according to Claim 12, **characterized in that** said surface is flat.

14. Use according to Claim 12, **characterized in that** said surface consists of a bead.

15. Use according to Claim 12, **characterized in that** it consists in carrying out assays to detect, in a liquid to be analysed, the presence of any biological molecule capable of attaching, via a specific binding site, to the support.

16. Use according Claim 12, **characterized in that** it consists in carrying out immunological assays to detect, in a liquid to be analysed, the presence of antibodies specific for a natural protein, for example the P24 protein, by specific binding, in the same way as with the natural protein, to at least one recombinant protein, for example the RH24 or R24 protein.
